# EUROPEAN PATENT APPLICATION

(11) **EP 0 974 650 A1**
(43) Date of publication of application: **26.01.2000**
(21) Application number: 98113653.4
(22) Date of filing: 22.07.1998
(51) Int. Cl.: C12N 15/12, C07K 14/47, C07K 16/22, A61K 38/18

(54) **Endothelial cell growth inhibitors**

(71) Applicant: University Of Ioannina/Medical School, 45110 Ioannino (GR); Schweigerer, Lothar, 45359 Essen (DE)
(72) Inventor: Fotsis, Theodore, 45500 Anatoli, Ioannina (GR); Schweigerer, Lothar, 45359 Essen (DE)
(74) Representative: Laudien, Dieter, Dr.

(57) **Abstract**

New proteins are provided which are characterized in that their expression is downregulated by the expression of the oncogen MYCN and in that they are capable of inhibiting endothelial cell growth under the proviso that they are not activin A.

## Description

### Field of the invention

The present invention relates to proteins characterized in that their expression is downregulated by the expression of the oncogen MYCN and in that they are capable of inhibiting endothelial cell growth. The invention also relates to pharmaceutical compositions and uses of said proteins. The invention further relates to antibody molecules directed against said novel proteins, processes for their preparation, pharmaceutical compositions and uses of said antibodies.

### Background of the invention

Cell growth in tissues is a strictly regulated process to avoid pathological and often malignant growth. For supporting growing cells with nutrients, new capillaries are generated from pre-existing vessels in a process called angiogenesis. Except in a few conditions such as wound healing, and the formation of corpus luteum, endometrium and placenta, angiogenesis is virtually absent in healthy adult organisms. These conditions of physiological angiogenesis represent ordered, tightly regulated and self-limited processes.

In certain pathological conditions, angiogenesis is dramatically enhanced and loses its self-limiting capacity. Pathological angiogenesis occurs during the development and progression of many diseases, such as rheumatoid arthritis, psoriasis, and diabetic retinopathy. Probably the most important manifestation of pathological angiogenesis is that induced by solid tumors. Well vascularized tumors expand both locally and by metastasis while avascular tumors do not grow beyond a diameter of 1 to 2 mm.

Angiogenesis is an essential part of the phenotypic repertoire that characterizes a successful tumor. It is often activated during early, preneoplastic stages in the development of a tumor and is subject to specific regulatory controls. In situ carcinomas may exist for months or years without neovascularization and remain limited to a small volume of few cubic millimeters. The angiogenic phenotype may be suddenly acquired by a small group of cancer cells (4-10%), finally leading to the formation of a solid, malignant tumor.

Mechanisms responsible for persistent angiogenesis are still unclear and certainly not the same in each disease. There is evidence that an imbalance between angiogenic factors and inhibitors, favoring the activity of angiogenic factors or the reduction or complete loss of inhibitors leads to angiogenesis.
The origin of angiogenic activation in malignant cells is unknown. Recent studies outline a relationship between genetic alteration during tumor progression and regulation of molecules that control angiogenesis. It was demonstrated that normal p53 regulates the expression of the angiogenesis inhibitors thrombospondin (TSP-1) (Dameron, K. M., Volpert, O. V., Tainsky, M. A. & Bouck, N. (1994) *Science* **265**, 1582-1584, Volpert, O. V., Stellmach, V. & Bouck, N. (1996) *Breast Cancer Res. Treat.* **36**, 119-126) and glioma-derived angiogenesis inhibitory factor (GD-AIF) (King, M. W. & Moore, M. J. (1994) *Nucleic Acids Res.* **22**, 3990-3996). Thus, loss or mutation of tumor suppressors at some stage of the process of neoplastic transformation leads to down-regulation of angiogenesis inhibitors and activation of neovascularization, which greatly contributes to the formation of solid, malignant tumors.

Little is known about the relationship between activation of oncogenes and initiation of angiogenesis. It was shown that activation of *ras* (Thompson, T. C., Southgate, J., Kitchener, G. & Land, H. (1989) *Cell* **56**, 917-930) and *int-2* (De Ines, C., Leynadier, D., Barasoain, I., Peyrot, V., Garcia, P., Briand, C., Rener, G. A. & Temple, C.,Jr. (1994) *Cancer Res.* **54**, 75-84) oncogenes does indeed induce angiogenesis. But molecules and mechanisms involved were not identified.
The MYCN oncogene also plays an important part in tumor progression activation and initiation of angiogenesis. Amplification of MYCN oncogene is a frequent event in advanced stages (III and IV) of human neuroblastomas (Brodeur, G. M., Seeger, R. C., Schwab, M., Varmus, H. E. & Bishop, J. M. (1984) *Science* **224**, 1121-1124), and is associated with enhancement of the malignant phenotype leading to unfavorable prognosis (Grady-Leopardi, E., Schwab, M., Ablin, A. & Rosenau, W. (1986) *Cancer Res.* **46**, 3196-3199) as well as enhanced vascularization of the tumors (Meitar, D., Crawford, S. E., Radmaker, A. W. & Cohn, S. L. (1996) J. *Clin. Oncol.* **14**, 405-414).

Because of the importance of angiogenesis in tumor progression and malignancy, there is a strong need for the characterization of molecules involved in the regulation of angiogenesis as well as the need for new tools for influencing angiogenesis.

Therefore, the problem underlying the present invention is to identify and characterize proteins regulated by the expression of the oncogen MYCN.

### Disclosure of the invention

The technical problem is solved by the embodiments characterized in the claims. The present invention provides new proteins characterized in that their expression is downregulated by the expression of the oncogene MYCN under the proviso that they are not activin A.

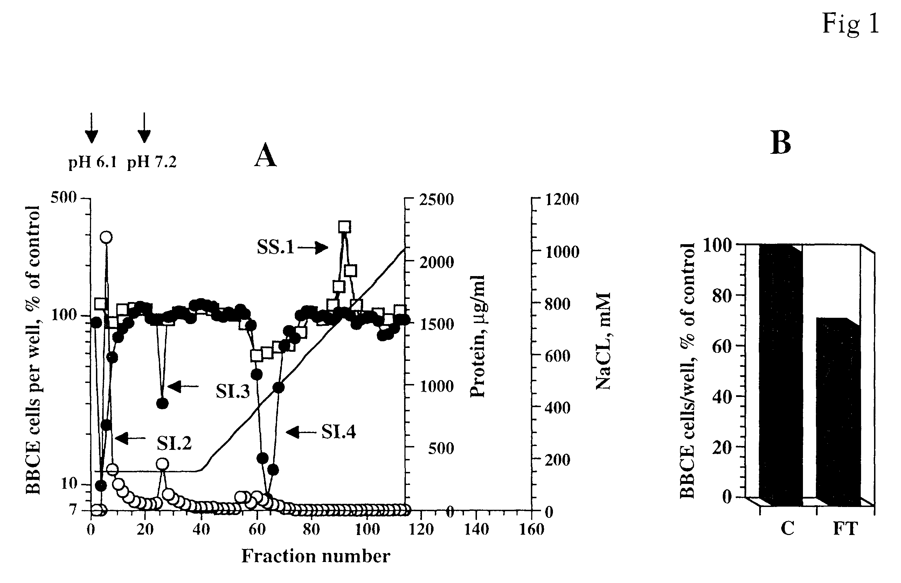

For the purpose of differentiating proteins regulated by the MYCN oncogene from MYCN-independent proteins, a neuroblastoma cell line (SH-EP) is compared to a neuroblastoma SH-EP cell line (WAC 2), which was stably transfected with the MYCN oncogene resulting in an increased expression of the oncogene by 100-fold in the transfected cells ((WAC 2), (Schweigerer, L., Breit, S., Wenzel, A., Tsunamoto, K., Ludwig, R. & Schwab, M. (1990) *Cancer Res.* **50**, 4411-4416). The WAC 2 transfected cells acquired an enhanced proliferative potential and, unlike the SH-EP 007 cells (the control transfectants), are able to form colonies in soft agar and induce well vascularized tumors in nude mice (Schweigerer (1990), same as above).
Modulators of endothelial cell proliferation were identified in both MYCN-overexpressing cells (WAC 2) and control cells (SH-EP 007). The capability of endothelial cell proliferation of the identified modulators was determined according to the assay described in example 4.
Four inhibitors (SI.1, SI.2, SI.3 and SI.4) and one stimulator (SS.1) of endothelial cell proliferation were identified in control cells. The endothelial cell modulators are named according to their origin (S = SH-EP 007, W = WAC 2) and abilities to either inhibit (I) or stimulate (S) capillary endothelial cell proliferation. All proteins were retained on the cation exchanger (see example 3) (Fig. 1A) except SI.1 which was found in the flow through (Fig. 1 B). The lower percent inhibtion of SI.1 is the result of insufficient enrichment due to the lack of adsorption on the exchanger. Concentration of the flow through revealed a comparable activity to the other inhibitors. All activities were well separated during cation exchange chromatography being eluted with buffers of different pH or salt concentration.

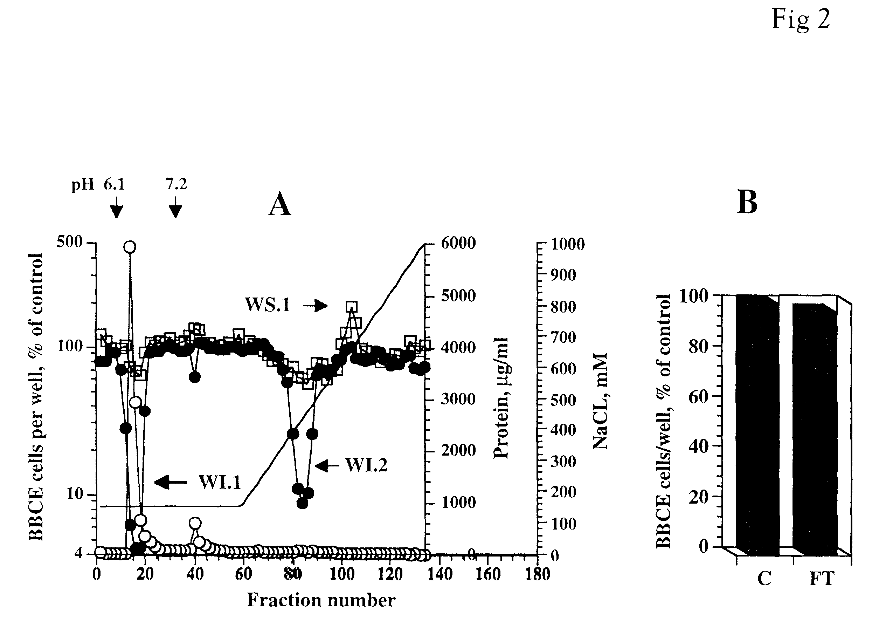

The profile of modulators of endothelial cell proliferation was different in the conditioned medium of the WAC 2 neuroblastoma cells overexpressing the MYCN oncogene (Fig. 2). The flow through was devoid of inhibitory activity (Fig. 2B) indicating the absence or very low expression of SI.1. Testing the activity after concentration of the flow through of the WAC 2 conditioned media confirmed the result. Elution of the exchanger with buffers at pH 6.0 exhibited an inhibitory activity (WI.1, Fig. 2A) which was eluted in a similar manner to that of SI.2 (Fig. 1A).

Subsequent testing revealed that this molecule (WI.1) was the enzyme arginine deiminase, a product of contaminating mycoplasma (Wilm, M., Shevchenko, A., Houthaeve, T., Breit, S., Schweigerer, L., Fotsis, T. & Mann, M. (1996) *Nature* **379**, 466-469). Treatment of the cells for mycoplasma contamination abolished this activity.

As a consequence, WAC 2 CM (CM = conditioned media) contained only one inhibitor (WI.2) and one stimulator (WS.1) of endothelial cell proliferation (Fig. 2A). These molecules were identified to be the same as SI.4 and SS.1 of the SH-EP 007 CM, respectively.

Accordingly, enhanced MYCN expression demonstrates strong down-regulation of three inhibitors (SI.1, SI.2, and SI.3) of endothelial cell proliferation. SI.3 was later identified as activin A (Mather, J.P., et al. Activins, inhibins and follistatins: Further thoughts on a growing family of regulators. Proc. Exp. Soc. Exp. Biol. Med. 215:209-222, 1997). This profile of angiomodulators was reproducible throughout several collections during the course of the study.

**Table 1**

| Comparison of Modulators in WAC-2 and SH-EP 007 cells | | |
|---|---|---|
| Control | MYCN | Identity |
| SH-EP 007 | WAC-2 | |
| SI.1 | - | novel |
| SI.2 | - | novel |
| SI.3 | - | activin A |
| SI.4 | WI.2 | identical to TGFβ1 |
| SS.1 | WS.1 | similar, possibly bFGF-related |

Therefore, in one embodiment the present invention relates to proteins whose expression is downregulated by the expression of the oncogene MYCN under the proviso that they are not activin A.
In a preferred embodiment, the invention relates to proteins of the present invention, characterized in that they are capable of inhibiting endothelial cell growth

### Identity of SS.1 and WS.1

The stimulators of endothelial cell proliferation found in conditioned media of neuroblastoma cells with normal (SH-EP 007) and enhanced MYCN expression (WAC 2) were eluted from cation exchangers with approx. 700 mM NaCI at pH 7.2 each (Fig. 1A and 2A). Characterization revealed that both molecules had heparin binding domains and were retained on Heparin-Sepharose columns. Both were eluted at high salt concentrations, SS.1 eluting at 1.4 M (Fig. 3A) and WS.1 at 1.9 M NaCI (Fig. 3B). These properties suggested a possible relation to bFGF. Neutralization experiments of the proliferative activity on endothelial cells of the stimulators with anti-bFGF antibodies demonstrated an inhibition of activity of about 50%. The antibody used did not cross-react with aFGF.

### Identity of SI.4 and WI.2

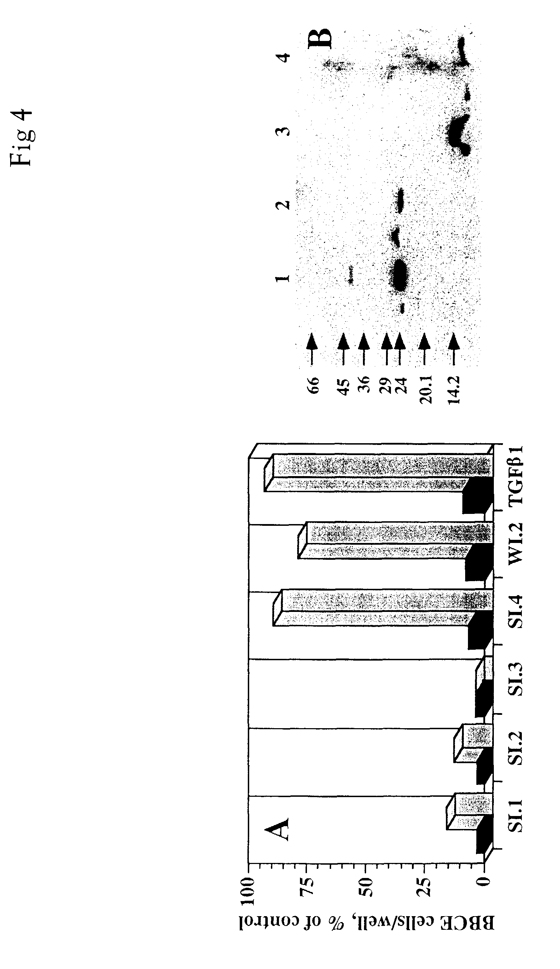

Conditioned media of neuroblastoma cells with normal (SH-EP 007) and enhanced MYCN expression (WAC 2) each contained an inhibitor of endothelial cell proliferation (SI.4 or WI.2, respectively) that was eluted from cation exchangers with buffers at pH 7.2 (440 mM NaCI, pH 7.2) (Fig. 1A and 2A). Chromatographic analysis revealed that both molecules were retained on Heparin-Sepharose columns, eluting at 800 mM NaCI. Both inhibitors were retained on chelating Sepharose columns loaded with copper; elution from the column was achieved with 78 mM imidazole. This data strongly indicated a possible identity to TGFβ1. This was confirmed when antibodies directed against TGFβ1 inhibited the bioactivity of the inhibitors (Fig. 4A), as well as recognized both inhibitors as homodimeric proteins of a molecular weight of 25 kDa (Fig. 4B). In conclusion, SI.4 and WI.2 are both identical to TGFβ1.

### Identity of SI.3

### SI.3 was identified by amino acid sequencing to be activin A.

The endothelial cell growth inhibitors SI.1 and SI.2 were detected in human neuroblastoma cells with normal (SH-EP 007) but not enhanced (WAC 2) MYCN expression. For further purification and characterization they were subjected to a series of chromatographies (see example 3). The molecules of the invention may be further purified. SI.1, for example, may be further processed by affinity chromatography on Con A-Sepharose, preparative native PAGE or preparative SDS-PAGE. After these steps the protein may be considered substantially pure but not yet entirely pure. It can be expected that a further HPLC step will lead to purification to homogeneity. Similar and other methods described in the state of the art and well known to any expert in the field of protein purification will also be suitable to purify SI.1 and/or SI.2.

The SI.1 molecule is characterized by its molecular weight. SI.1 exhibits a molecular weight of about 47 ±4kD when determined by gel filtration (when applying a method according to the procedure of example 3). The term "about" as used in this description in context with technical data is defined as including the complete possible range of absolute values that may be demonstrated when executing the technical procedures required for obtaining such data with due diligence. The possible range of absolute values is statistical and dependent on the technical devices, reagents, manual and technical techniques and on the inherent properties of the substances under investigation. To determine identity of the proteins of the present invention with other proteins, it is therefore necessary to subject both proteins to be compared to exactly the same reagents, technical setup and manipulations. The molecular weight by gel filtration is not always accurate. Several proteins are known which elute in elution volumes that differ from their actual molecular weight. SI.1 displays a molecular weight, when measured by SDS-PAGE, of about 37 ±3kD. Said latter molecular weight was determined by checking the activity after renaturing the protein following preparative SDS-PAGE without mercaptoethanol. SI.1 may therefore consist of subunits or a single unit. The limitations of the methods for the determination of molecular weights of proteins also hold true for the protein Sl.2. The proteins of the invention may be glycosylated or deglycosylated and will then display different molecular weights, but are still included in the invention as long as they are downregulated by MYCN or/and capable of inhibiting endothelial cell growth.
In a particular embodiment the invention relates to proteins according to the invention that have a molecular weight of about 47 kD when determined by gel filtration.
In chromatofocusing SI.1 presents two peaks with isolelectric points of about 8.0±0.5 and 6.2 ±0.4. The uncertainty experienced with molecular weight determinations also applies to isoelectric point measurement by chromatofocussing. Because of the low salt concentration and the possibility of non-specific binding to other proteins many proteins exhibit multiple isoelectric points. There is also the possibility of proteolysis during the purification procedure resulting in multiple or erroneous isoelectric points. These reservations with respect to chromatofocussing hold true for all proteins and particularly for SI.1 and SI.2.
In a particular preferred embodiment the present invention relates to proteins according to the invention characterized in that they present two peaks with isoelectric points of about 8.0 and 6.2 in chromatofocussing.
SI.1 can be further characterized by its binding affinities. SI.1 has affinity neither to copper nor to heparin as demonstrates by its inability to bind to Chelating-Sepharose columns loaded with copper and Heparin-Sepharose. Therefore, in a particular more preferred embodiment, the present invention relates to proteins according to the invention characterized in that they have no affinity to copper or heparin.

Both isoelectric forms of SI.1 exhibit strong affinity to Con A-Sepharose columns indicating that they are glycosylated and probably derived from the same molecule. Therefore, in a particular more preferred embodiment, the present invention relates to proteins according to the invention characterized in that they are glycosylated. The SI.1 molecule is heat and acid stable and does not lose its inhibitory activity even after treatment at 90°C for 10 min or after treatment with 0.1 M HCI. Therefore, in a particular more preferred embodiment, the present invention relates to proteins according to the invention characterized in that they are heat stable and do not lose their inhibitory activity after treatment at 90°C for 10 minutes. Also, in a particular more preferred embodiment, the present invention relates to proteins according to the invention characterized in that they are acid stable and do not lose their inhibitory activity after treatment with 0.1 M HCl.

In a particular most preferred embodiment, the present invention relates to proteins according to the invention characterized in that:
- they have a molecular weight of about 47 kD when determined by gel filtration,
- they present two peaks with isoelectric points of about 8.0 and 6.2 in chromatofocussing,
- they have no affinity to copper or heparin,
- they are glycosylated,
- they are heat stable and do not lose their inhibitory activity after treatment at 90°C for 10 minutes,
- they are acid stable and do not lose their inhibitory activity after treatment with 0.1 M HCI.

The SI.2 molecule displays molecular weights of about 47±4kD and 29±2.5kD when determined by gel filtration (when applying a method according to the procedure of example 3). Therefore, in a particular embodiment the present invention relates to proteins according to the invention characterized in that the proteins have two molecular weights of about 47 and 29 kD when determined by gel filtration.
Heterogeneity of SI.2 is also apparent during chromatofocusing where SI.2 is resolved to three peaks, the major peak being that with an isoelectric point of about 5.0±0.3. In a more particular embodiment the present invention relates to proteins according to the invention characterized in that the proteins present two minor peaks and a main peak with an isoelectric point of about 5.0 in chromatofocussing.

The SI.2 molecule is not glycosylated and has no affinity to copper or heparin as demonstrated in the same manner as above for SI.1. In a more particular embodiment the present invention relates to proteins according to the invention characterized in that the proteins have no affinity to copper or heparin. In a more particular embodiment the present invention relates to proteins according to the invention characterized in that the proteins are not glycosylated.

SI.2 is acid stable. Therefore, in a more particular embodiment the present invention relates to proteins according to the invention characterized in that the proteins are acid stable and do not lose their inhibitory activity after treatment with 0.1 M HCl.

SI.2 seems less stable than SI.1 because the inhibitory activity is lost after one week storage at +4°C. On the other hand, heat treatment at 60°C for 60 min does not reduce the activity of the protein. Therefore, in a more particular embodiment the present invention relates to proteins according to the invention characterized in that the proteins are heat stable and do not lose their inhibitory after treatment at 60°C for 60 minutes.
In a particular most preferred embodiment, the present invention relates to proteins according to the invention characterized in that:
- the proteins have two molecular weights of about 47 and 29 kD when determined by gel filtration,
- they present two minor peaks and a main peak with an isoelectric point of about 5.0 in chromatofocussing,
- they have no affinity to copper or heparin,
- they are not glycosylated,
- they are heat stable and do not lose their inhibitory activity after treatment at 60°C for 30 minutes,
- they are acid stable and do not lose their inhibitory activity after treatment with 0.1 M HCI.

The properties of the SI.1 and SI.2 molecules are summarized in Table 1 and compared to those of the known inhibitors of endothelial cell proliferation. Based upon these properties, both inhibitors are novel molecules.

**Table 1.**

| Comparison of the physiochemical properties of the isolated with those of know inhibitors of endothelial cell proliferation *in vitro* | | | | | | |
|---|---|---|---|---|---|---|
| | Mr, kD | Isoelectric point | Glycosylation | Acid-stability | Heat-stability | Heparin-binding |
| IL-1α | 17 | 5.0 | + | + | + | - |
| IL-1β | 17 | 7.0 | + | + | - | - |
| PF-4 | 38.4 | 7.6 | | | | + |
| IFN-γ | 20-25 | 8.6-8.7 | + | - | + | - |
| Thrombospondin | > 140 | | | | | |
| TIMP-1 | 30 | | + | + | + | - |
| TIMP-2 | 21-28 | | ± | + | + | - |
| Angiostatin | 38 | | | | | + |
| TGFβ1 § | 25 | 4.0 | - | + | + | + |
| TNFα § | 51 | 4.0 | - | | | - |
| TNFβ § | 20-25 | 5.8 | ? | | | - |
| SI.1 | 47 | 8.0/6.2 | + | + | + | - |
| SI.2 | 32/47 | 5.0 (main) | - | + | + | - |

| | | | | | | |
|---|---|---|---|---|---|---|
| § These substances administered *in vivo* stimulate angiogenesis via indirect mechanisms | | | | | | |

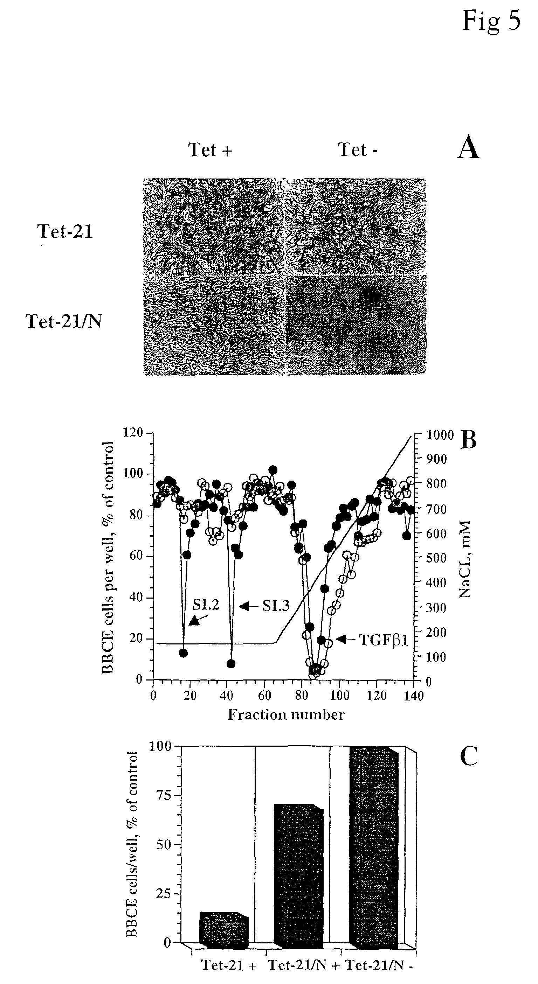

SI.1 and SI.2 are proteins found in conditioned medium (see examples 1 and 2) of the SH-EP 007 cells indicating that enhanced expression of MYCN in WAC 2 cells considerably downregulates the expression of these inhibitors. To eliminate any possibility that this regulation is caused from additional genetic changes of long term culture an inducible eucaryotic expression system based on the tetracycline repressor is used (Gossen, M. & Bujard, H. (1992) *Proc. Natl. Acad. Sci. USA* **89**, 5547-5551, Gossen, M., Freundlieb, S., Bender, G., Müller, G., Hillen, W. & Bujard, H. (1995) *Science* **268**, 1766-1769). Immunodetection, Northern analysis and immunoprecipitation experiments revealed a low detectable level of MYCN expression in Tet-21/ N cells in the presence of tetracycline. This indicates that tetP can not be shut off completely by tetracycline (Lutz, W., Stöhr, M., Schürmann, J., Wenzel, A., Löhr, A. & Schwab, M. (1996) *Oncogene* **13,** 803-812). Removal of tetracycline from these cells results in a ten fold increase of the MYCN protein and fifteen fold increase in the level of MYCN mRNA (Lutz et al. (1996), same as above). In the respective precursor clone Tet-21, which was employed as control, MYCN expression could not be detected as well as in the untransfected SH-EP cells. The leaky expression of MYCN, even in the presence of tetracycline, is correlated with the morphology of the cells *in vitro* (Fig. 5A). The Tet-21 cells, which do not express MYCN, exhibit the typical morphology of SH-EP both in the presence and absence of tetracycline (upper panel, Fig. 5A). The Tet-21/ N cells showed altered morphology and higher cell density even in the presence of tetracycline (lower left panel, Fig. 5A), whereas removal of tetracycline resulted in strong enhancement of the malignant phenotype of the cells and generation of foci (right lower panel, Fig. 5A).

Using this inducible system, conditioned media of the Tet-21 and Tet-21/ N cells were examined for the presence of SI.1 and SI.2 endothelial cell growth inhibitors. When conditioned medium from Tet-21 cells are subjected to cation exchange chromatography, considerable activity of SI.1 is observed in the flow-through. In contrast, SI.1 activity is reduced in Tet/N supernatants, markedly in the presence and completely in the absence of tetracycline (Fig. 5B). In accordance with this data Tet-21 conditioned media contain considerable quantities of SI.2 and SI.3 which are dramatically down-regulated in media from Tet-21/ N cells even in the presence of tetracycline (Fig. 5C). This data demonstrates that even a weakly enhanced expression of MYCN oncogene, resulting from leaky expression of the transfected MYCN cDNA construct, is sufficient to cause downregulation of the SI.1 and SI.2 as well as SI.3. In contrast, TGFβ1 is not downregulated. (Fig. 5C). The term "downregulated" as used herein refers to a decrease in concentration of a particular substance under investigation, e.g. SI.1, SI.2, by at least a factor of 5, wherein the decrease in concentration is directly correlated with the expression of the oncogene MYCN.
The present invention demonstrates that MYCN-regulated angiomodulators play an important role in the initiation of endothelial cell growth and angiogenesis and thus contribute to the tumor progression caused by the amplification of the MYCN oncogene in neuroblastoma. Rather than activating the production of angiogenesis inducers, the MYCN oncogene downregulates the expression of inhibitors of angiogenesis. The genotypic repertoire that characterizes the successful tumor cell has to overcome the strong inhibitory influences by down-regulating the highly and constitutively expressed inhibitors of angiogenesis in normal cells.

As used herein, the term "protein capable of inhibiting endothelial cell growth" refers to a protein exhibiting an inhibitory biological activity when assessed in a cell proliferation assay, such as described in an exemplary manner in example 4.

An expert in the field of protein chemistry can easily modify and derive new proteins from the proteins disclosed herein, that will retain the biological inhibitory activity of the original proteins. When one of the numerous methods available from the present state of the art is applied to the disclosed proteins, there is a more than reasonable expectation of success that an expert arrives at a functional derivative. As used herein, a "functional derivative" of a protein of the invention is a protein which possesses the biological activity (either functional or structural) that is substantially similar to the biological activity of the original protein. A molecule is said to be "substantially similar" to another molecule if both molecules have substantially similar structures, e.g. 80 % amino acid sequence homology, and if both molecules possess a similar biological activity. The "functional derivatives" of the proteins of the invention include fragments, variants, chemical derivatives or fusion proteins of said proteins. The term "fragment of a protein of the present invention" is meant to refer to any polypeptide subset of that molecule. The term "variant of a protein of the present invention" is meant to refer to a protein substantially similar in structure to either the entire molecule, or to a fragment thereof, provided that the variant has at least a biological activity similar to that of the proteins of the invention. A variant of a protein of the invention may differ from a protein of the invention by the substitution, deletion or addition of one or more amino acids, preferably 1 to 10 amino acids. A "chemical derivative of a protein of the present invention" is a molecule which has been derived from a protein disclosed herein by a chemical reaction, e.g. iodination, acetylation, or linkage to a radioisotope or toxin. A "fusion protein of a protein of the present invention" is a protein which has been generated by recombinant expression of all or a part of the gene for the proteins of the present invention fused to all or part of another gene or nucleic acid containing in-frame coding information.
Therefore, a further aspect of the present invention includes functional derivatives of any of the proteins of the invention, wherein said functional derivatives are variants, fragments, chemical derivatives, or fusion proteins of said protein.

Elucidation of the molecules and mechanisms involved in the initiation of angiogenesis in solid tumors is currently of great scientific and clinical interest. Beyond the goal of clarifying the complex process of angiogenesis such knowledge will provide targets for therapeutic intervention and open the horizon for an anti-angiogenic approach for the treatment of pathological conditions such as solid tumors. The list of angiogenesis inhibitors is constantly increasing and there are already nine compounds in clinical trials as anti-angiogenic agents (Hanahan, D. & Folkman, J. (1996) *Cell* **86**, 353-364)

One aspect of the present invention relates to pharmaceutical compositions containing one or more proteins of the present invention or pharmaceutically acceptable salts thereof.
The proteins of the invention have demonstrated their ability to inhibit cell proliferation and endothelial cell growth. Therefore, in a preferred embodiment the present invention relates to pharmaceutical compositions for the treatment of pathological cellular growth, more particular pathological endothelial growth. In a most preferred embodiment the present invention relates to pharmaceutical compositions for the treatment of pathological angiogenic growth.

Given the information of the present invention, especially the information on how to obtain proteins according to the invention, the expert can now produce antibodies specific for said proteins, or functional derivatives thereof, according to standard procedures (Catty, D. (ed.). 1989. Antibodies - a practical approach. Vols. 1 and 2. IRL press, Oxford, especially vol. 1, chapters 2, 3, 4). For use as an antigen, for example, a protein of the invention or part thereof can be used in an immunization protocol, optionally linked to a carrier. The protein or derivative thereof can then be used in an immunization scheme. Specific antibodies or - in the case of monoclonal antibodies - hybridomas which produce specific antibodies can then be selected by appropriate methods (Wunderlich, D., A. Lee, R. P. Fracasso, D. V. Mierz, R. M. Bayney, T. V. Ramabhadran. 1992. Use of recombinant fusion proteins for generation and rapid characterization of monoclonal antibodies. J. Immunol. Methods **147**:1-11.). Antibodies may either be monoclonal or polyclonal. Instead of an intact immunglobulin, fragments of immunglobulins may be used, for example Fab' or F(ab)₂ fragments. The production of recombinant antibodies or antibody fragments, chimeric antibodies, humanised antibodies, bispecific antibodies or single-chain antibodies for a given antigen are state of the art. Antibodies may be coupled to other agents, e.g. radioisotopes or toxins. Antibodies or functional derivatives of said antibodies specific for the proteins of the invention are useful tools for studying the mechanism of cellular events relating to endothelial growth and/or angiogenesis in normal and/or pathologically altered cells.

In a most preferred embodiment, the present invention relates to antibody molecules specific for the proteins of the present invention, which are polyclonal antibodies, monoclonal antibodies, complete immunglobulines or fragments thereof, the Fab' or F(ab)₂ fragments of immunglobulines, recombinant antibodies or recombinant antibody fragments, recombinant single-chain antibodies (scFv), chimeric, bispecific or humanised antibodies.

Such antibodies can also be used to neutralise or impair the transmission of the biological signal that inhibits angiogenesis and/or endothelial cell growth. In a preferred embodiment the present invention relates to antibody molecules characterized in that said antibodies neutralize the biological activity of the proteins of the invention.

In a further aspect the invention relates to processes for preparing antibodies specific for the proteins of the present invention. In one embodiment the present invention relates to processes for preparing polyclonal antibodies specific for said proteins, characterized in that suitable host animals are immunized with said proteins and polyclonal antibodies are isolated from said host animal.

In another embodiment the present invention relates to processes for preparing monoclonal antibodies specific for proteins according to the invention, characterized in that suitable host animals are immunized with said proteins, B-lymphocytes of these host cells are fused with myeloma cells, and a hybrid cell line secreting the monoclonal antibody is subcloned and cultivated.

In a further embodiment the present invention relates to processes for preparing hybrid cell lines characterized in that they secrete monoclonal antibodies specific for the proteins of the invention.

Another aspect of the invention relates to pharmaceutical compositions comprising an antibody molecule according to the invention or pharmaceutically acceptable salts thereof.
The antibodies according to the present invention can neutralize the endothelial growth inhibiting biological activity of the disclosed proteins. They will therefore prove useful agents for the treatment of pathological cellular growth conditions.
In one embodiment the present invention relates to pharmaceutical compositions for the treatment of pathological cellular growth conditions, characterized in that normal endothelial cell growth is absent or pathologically reduced.
In a preferred embodiment the present invention relates to pharmaceutical compositions for the treatment of pathological conditions characterized in that normal angiogenic growth is absent or pathologically reduced.
In a most preferred embodiment the present invention relates to pharmaceutical compositions characterized in that the pathological condition resulted from a wound.

Pharmaceutical compositions comprising proteins or antibodies of the invention can be used to treat an animal or human body. In an animal or human body, it can prove advantageous to apply the pharmaceutical compositions of the invention systemically, locally or topically to the tissue or organ of concern, depending on the type and origin of the disease or problem treated, e.g. a tumor or a wound. For example, a systemic mode of action is desired when different organs or organ systems are in need of treatment as in e.g. tumors that are diffuse or difficult to localise. A local mode of action would be considered when only local manifestations of neoplastic action or wounds are expected, such as, for example local tumors.
The proteins and/or antibody proteins of the present invention may be applied by any enteral or parenteral route of application known to the expert. The dose-range for the proteins and/or antibody proteins of the present invention lies between 0.001 and 100 mg per kg of a human or animal body in need of such treatment. For systemic application, the intravenous, intravascular, intramuscular, intraarterial, intraperitoneal, oral, or intrathecal route are preferred. A more local application can be effected subcutaneously, intracutaneously, intracardially, intralobally, intramedullarly, intrapulmonarily or directly in or near the tissue to be treated (connective-, bone-, muscle-, nerve-, epithilial tissue). Depending on the desired duration and effectiveness of the treatment, pharmaceutical compositions comprising proteins or antibodies of the invention may be administered once or several times, also intermittently, on a daily basis for several days, weeks or months and in different dosages.
For preparing suitable antibody or protein preparations for the applications described above, the expert may use known injectable, physiologically acceptable sterile solutions. For preparing a ready-to-use solution for parenteral injection or infusion, aquous isotonic solutions, such as e.g. saline or corresponding plasmaprotein solutions are readily available. The pharmaceutical compositions may be present as lyophylisates or dry preparations, which can be reconstituted with a known injectable solution directly before use under sterile conditions, e.g. as a kit of parts. The final preparation of the protein or antibody compositions of the present invention are prepared for injection, infusion or perfusion by mixing purified antibodies according to the invention with a sterile physiologically acceptable solution, that may be supplemented with known carrier substances or/and additives (e.g. serum albumin, dextrose, sodium bisulfite, EDTA)
The amount of the antibody or protein applied depends on the type and gravity of the disease condition.

Since the antibodies according to the invention present useful pharmaceutical, diagnostic, preparative and analytical tools, it is of great convenience to any expert to obtain monoclonal antibodies specific for the proteins of the invention in close confinement with other reagents or tools necessary to perform the deed of interest. Such reagents can include liquid or solid reagents such as buffer solutions, or technical material such as flasks or syringes. In a further aspect the invention relates to a kit of parts containing in close confinement one or more carrier means, characterized in that one carrier means contains a monoclonal antibody according to present invention for determining a protein of the present invention.

In another aspect the present invention refers to the use of antibodies specific for the proteins of the present invention.

Polyclonal and monoclonal antibodies are useful tools for purifying their targets, for example, by affinity chromatography, especially when bound to solid support. In one embodiment the present invention relates to the use of antibodies according to the invention for purifying the proteins of the present invention.

Such antibodies are also useful as diagnostic and/or research tools for quantifying the proteins of the invention *in vivo* or *in vitro.* In a preferred embodiment the present invention relates to the use of antibodies according to the invention for qualitatively and/or quantitatively determining the proteins of the invention *in vitro* and *in vivo.*

In a preferred embodiment the present invention relates to the use of antibodies according to the invention for neutralizing the activity of the proteins of the invention *in vitro* and *in vivo.*

In a preferred embodiment the present invention relates to the use of antibodies according to the invention for activating and/or enhancing cell growth, more particular endothelial cell growth *in vitro* and *in vivo.*

In a preferred embodiment the present invention relates to the use of antibodies according to the invention for activating and/or enhancing angiogensis *in vitro* and *in vivo.*

In a further aspect the present invention refers to the use of the proteins of the present invention.

In a preferred embodiment the present invention relates to the use of the proteins of the invention for inhibiting cell growth, more particular endothelial cell growth *in vitro* and *in vivo.*

In a more preferred embodiment the present invention relates to the use of the proteins of the invention for inhibiting angiogenesis *in vitro* and *in vivo.*

In a more preferred embodiment the present invention relates to the use of the proteins of the invention for inhibiting pathological cell growth, more particular pathological endothelial cell growth *in vitro* and *in vivo.*

In a more preferred embodiment the present invention relates to the use of the proteins of the invention for the preparation of a pharmaceutical composition.

In a more preferred embodiment the present invention relates to the use of the antibodies according to the invention for the preparation of a pharmaceutical composition.

In a more preferred embodiment the present invention relates to the use of the proteins of the invention for the preparation of the antibodies according to the invention.

### Figure legends

**Fig 1.** *Cation exchange chromatography of the SH-EP 007 conditioned medium.*
**A.** The graph shows the elution profile of the chromatography described in example 9. The salt concentration of the eluting buffer (-), the protein content (o) and the activity on endothelial (BBCE) cell proliferation (•) of the eluted and consequitively numbered fractions are depicted in the graph.
**B.** The activity of the flow through (FT) on endothelial (BBCE) cell proliferation is compared to that of a control sample (C).

**Fig. 2.** Cation exchange chromatography of the WAC 2 conditioned medium.
**A.** The graph shows the elution profile of the chromatography described in example 10. The salt concentration of the eluting buffer (-), the protein content (o) and the activity on endothelial (BBCE) cell proliferation of the eluted and consequitively numbered fractions are depicted in the graph.
**B.** The activity of the flow through (FT) on endothelial (BBCE) cell proliferation is compared to that of a control sample (C).

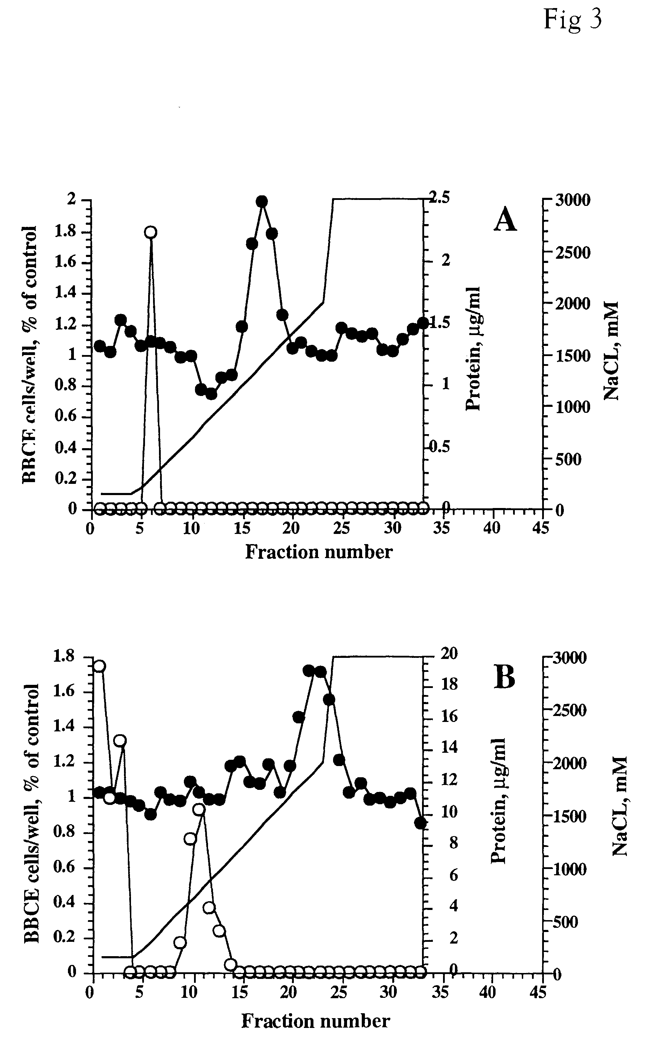

**Fig. 3.** *Heparin - Sepharose chromatography of SS.1 (**A**) and WI.2 (**B**)*
The graph shows the elution profile of the chromatographies described in example 11. The salt concentration of the eluting buffer (-), the protein content (o) and the activity on endothelial (BBCE) cell proliferation of the eluted and consequtively numbered fractions are depicted in the graph.

**Fig. 4.** *Identification of TGFβ1*
**A.** This graph depicts the inhibtory activity on endothelial cell proliferation of the tested substances as described in example 12. The activity is expressed as % control, referring to the endothelial cell number after addition of bFGF and buffer only.
**B.** The picture depicts the immunodetection of partially purified fractions of SI.4 after electrophoresis on 13.5 % polyacrylamide gel as described in example 12.
SI.4 is shown in lane 2 without β-mercaptoethanol and with β-mercaptoethanol in lane 4. Lanes 1 (without β-mercaptoethanol) and 3 (with β-mercaptoethanol) show TGFβ1. The sizes (kD) of the different bands of the marker are indicated with arrows on the left side.

**Fig. 5.** *Inducible expression of MYCN in neuroblastoma cells; effect on the expression of the inhibitors of endothelial cell proliferation*
**A**) This figure shows the effect of MYCN expression on the morphology and transformation level of neuroblastoma cells in the presence (+) or absence of tetracyclin (-). Tet-21: neuroblastoma cells tranfected with transactivator only. Tet-21/ N: neuroblastoma cells transfected with transactivator and MYCN cDNA.
**B**) This graph shows cation exchange chromatography of Tet-21 + (•) and Tet-21 / N + (o) conditioned media. Cation exchange chromatography and activity evaluation on endothelial cell proliferation as in Fig 1.
**C**) This graph shows the antiproliferative activity of concentrated (20 x) flow through after cation exhange chromatography of Tet-21 (+), Tet-21/N (+), and Tet-21/N (-) conditioned media for detection of SI.1 activity.

### Examples

### Example 1 Cell culture

Unless otherwise indicated dishes and medium used for tissue culture, and human recombinant bFGF were from sources mentioned earlier (16).

Endothelial cells derived from brain capillaries (BBCE) and adrenal cortex (ACE) were kindly provided by D. Gospodarowicz (University of California, San Francisco, CA.). Endothelial cells derived from bovine aorta (BAE) were obtained from Dr. P. Nawroth (Department of Internal Medicine, University of Heidelberg, Germany). Endothelial cell from human umbilical vein (HUVE) were a gift from Dr. J. Grulich-Henn (Children's Hospital, University of Heidelberg, Germany). BBCE, BAE, ACE and HUVE cells were maintained in Dulbecco's modified Eagle medium (DMEM) with low glucose concentration (1000 mg/ l), 10% newborn calf serum, glutamine (2 mmol/l), and antibiotics. Cultures received bFGF (2.5 ng/ml) every other day until confluent.

Tumor cells from human neuroblastoma (SH-EP) and their MYCN oncogene stable transfectants (WAC 2) were maintained in RPMI 1640 medium with 10% newborn calf serum and antibiotics. In the cultures of WAC 2 cells the antibiotic geneticin (200 µg/ml) was also added for selection.

### Example 2 Collection and initial processing of the conditioned media (CM)

The SH-EP 007 and WAC 2 cells were inoculated in square dishes (25 x 25 cm, Nunc, Roskilde, Denmark) and allowed to become confluent in the medium mentioned above. Afterwards the cultures were washed with PBS, and serum free medium (RPMI 1640 plus formulation consisted from insulin (5 µg/ml), transferin (5 µg/ml) and selenium (5 µg/ml) (Sigma, St. Louis, USA) was added. After 48 hours the supernatant was collected, centrifuged and stored at -80°C. Fresh serum free medium was added, and the same process was repeated. After two collections, medium supplemented with serum was added to strengthen the cells. Following 72 hours, serum free medium was added again, and the process was repeated as above. These cycles of culture in serum free medium alternated by culture in medium with serum was repeated until signs of exhaustion of the culture were evident by microscopy, usually after 4 to 6 weeks. Then, new cultures were initiated, and the whole process was repeated until approx. 100 liters of conditioned medium was collected from SH-EP 007 and WAC 2 cells.

To facilitate chromatographic analysis, the conditioned medium was concentrated 20 fold using ultrafiltration through membranes of 10.000 Mr cut off (Pellicon system, Millipore, Bedford, USA). The concentrated conditioned medium was acidified by adding 50 mM acetic acid buffer, pH 4.8. The acidified conditioned medium was allowed to stand at room temperature for 2 hours, followed by standing for 12-16 hours at 4°C. Before application onto cation exchangers the conditioned medium was centrifuged (10.000 g, 60 min, 4°C).

### Example 3 Chromatography

Cation exchange chromatography of conditioned media was carried out by applying the concentrated and acidified medium onto either a S-Sepharose High Performance (SSHP) (20 ml bed volume) or a S-Sepharose Fast Flow (SSFF) (180 ml bed volume) cation exchangers (Farmacia, Upsala, Sweden) equilibrated in 50 mM acetate buffer, pH 4.8 + 150 mM NaCI + 0.1 % CHAPS. The choice depended on the volume of the starting aliquot of the conditioned medium. Following application the column was eluted with i) 3 bed volumes of the equilibration buffer (not shown in the figures), ii) 5 bed volumes of 50 mM MES buffer, pH 6.1 + 150 mM NaCI + 0.1% CHAPS, iii) 5 bed volumes of 50 mM Tris-HCI buffer, pH 7.2 + 150 mM NaCI + 0.1 % CHAPS, and finally iv) by 15 bed volumes of a linear gradient of buffer iii up to a salt concentration of 1 M NaCI. The flow rate was adjusted to 60 cm/h.

Gel filtration chromatography were carried out using either Sephacryl - 100 HR or Superdex 200 columns (Pharmacia, Upsala, Sweden) (175 ml bed volume). The columns were equilibrated in 50 mM Tris-HCI, pH 7.2 + 1 m NaCI + 0.1% CHAPS + 1M Urea. Samples were applied in approx. 2 ml of the equilibration buffer, and elution was carried out with 1.2 bed volumes of equilibration buffer. Both application and elution were performed using reverse flow. The flow rate was adjusted to 15 cm/h whereas the fraction volume was 1.5 ml.

Heparin - Sepharose chromatography (Pharmacia, Upsala, Sweden) was carried out in 1 ml columns (0.8 cm diameter) equilibrated in 50 mM Tris-HCL, pH 7.2 + 150 mM NaCI + 0.1% CHAPS. Following application of the sample in the equilibration buffer, the column was eluted with i) 5 bed volumes of equilibration buffer, ii) linear gradient consisted from 10 bed volumes of equilibation buffer and 10 bed volumes of 50 mM Tris-HCI, pH 7.2 + 2 M NaCI + 0.1% CHAPS, and iii) 10 bed volumes of 50 mM Tris-HCI, pH 7.2 + 3 M NaCI + 0.1% CHAPS. A flow rate of 12 cm/h were used, and fractions of 1 ml volume were collected.

Con A - Sepharose chromatopgraphy (Pharmacia, Upsala, Sweden) was performed in columns of 5 ml bed volume (1.1 cm of diameter). Equilibration was carried out win 50 mM Tris-HCI, pH 7.2 + 500 mM NaCI + 0.1% CHAPS + 1 mM CaCl₂ + 1 mM MnCl₂. The sample was applied in the equilibration buffer. Elution was carried out using i) 3 bed volumes of equilibration buffer, ii) linear gradient consisted from 5 bed volumes of equilibration buffer and 5 bed volumes of equilibration buffer plus 200 mM a-methyl mannopyranoside, and iii) 5 bed volumes of equilibration buffer plus 500 mM a-methyl mannopyranoside. The fraction size was 1.5 ml whereas the flow rate 12 cm/h.

Metal chelation chromatography was carried out in small columns of 1.2 ml bed volume (0.8 cm diameter). The column (Chelating Sepharose Fast Flow, Pharmacia, Upsala, Sweden) was first loaded with copper by washing it with 10 bed volumes of 50 mM CuCl₂. Following loading of the metal, the column was equilibrated in 50 mM Tris-HCl, pH 7.2 + 500 mM NaCI + 0.1% CHAPS. The sample was applied in the same buffer. The column was then eluted with i) 3 bed volumes of equilibration buffer, ii) linear gradient consisted from 5 bed volumes of equilibration buffer and 5 bed volumes of equilibration buffer plus 100 mM imidazole, iii) 5 bed volumes of equilibration buffer plus 100 mM imidazole, and iv) 5 bed volumes of equilibration buffer plus 50 mM EDTA. The flow rate was adjusted to 12 cm/h, and fractions of 1 ml volume were collected.

Chromatofocusing was carried out in the following way. The active fractions from the previous column were concentrated and diafiltrated using ultrafiltration (YM 10 membranes, Amicon 50-ml cell). The final volume was 9 ml in 25 mM imidazole-HCL, pH 7.4 + 0.1% CHAPS. After application onto a PBE 94 column (Pharmacia, Upsala, Sweden) (20 ml bed volume equilibrated in the same buffer) elution was carried out using 14 bed volumes of polybuffer (Pharmacia) adjusted to pH 4.0 with HCL. Acidic compounds were eluted with 5 volumes of 0.1 M acetic acid + 1 M NaCI + 0.1% CHAPS. The flow rate was 30 cm/h. Fractions of approx. 2.5 ml were collected and aliquots were tested for protein content and inhibitory activity on endothelial cell proliferation. Columns with pH gradient from 8 to 5 were also used.

### Example 4 Cell proliferation assays

Stock cultures were trypsinized, cells were adjusted to a density of 5 x 10³ per ml in their respective media, and seeded in 1 ml aliquots per well into 12-well cluster dishes. After 16 h, wells received either 10 µl aliquots of fractions only (stimulatory assay), or 10 µl aliquots of fractions plus bFGF (2.5 ng/ml) (inhibitory assay), and these treatments were renewed every other day. Controls included wells treated either with buffer only or with buffer plus bFGF. Cells were counted at day 6 with a Coulter particle counter. Unless otherwise indicated, values of cell densities represent the means of duplicate determinations which varied by less than 10% of the mean.

### Example 5 Neutralization experiments

Aliquots of all partially purified inhibitors from SH-EP 007 and WAC 2 conditioned media were incubated with either chicken immunoglobulin or with chicken anti-TGFβ1 antibody (2.7 pg/ml) (R+D). Following incubation at room temperature for 30 min. the samples and controls were tested for inhibtory activity on endothelial cell proliferation as described above. The same was carried out using anti-bFGF antibodies raised in rabbits.

### Example 6 Immunodetection

Aliquots from partially purified fractions of SI.4 were electrophoresed on 13.5 % polyacrylamide gel with and without mercaptoethanol. Gels were transfered directly to nitrocellulose (ECL, Amersham, UK) using liquid blotting. The membraned was stained with Ponceau S (Sigma, St. Louis, USA) for detection of proteins and checking of the loading. Immunodetection was carried out using rabbit anti-TGFβ1 antibody (1:1000, R+D) as the primary antibody followed by incubation with an anti-rabbit IgG labelled with peroxidase (Amersham, UK). Detection was carried out using an enhanced chemiluminescence system (ECL, Amersham, UK) following the instructions of the manufacturer.

### Example 7 Stability in heat and acid conditions

Partially purified fractions (25 µl), to which 25 µl of 0.2% gelatin in PBS had been added, of SI.1, SI.1 and SI.3 were heated at 4°C, 37°C and 60°C for 60 min and at 90°C for 10 min. Following heating, the samples and the corresponding controls were centrifuged and 10 µl of the supernatants was tested for inhibitory activity as described above.

Aliquots of partially purified fractions of SI.1, SI.2, and SI.3 were brought to 0.1 M acetic acid or 0.1 M HCI concentrations. Following 60 min incubation the samples and relevant controls were neutralized by NaOH, and aliquots were tested for activity as described above.

### Example 8 Inducible expression of MYCN in neuroblastoma cells

All plasmids, cell culture, transfection and selection procedure were published earlier. Briefly, in the first co-transfection the pUHD15-1 plasmid containing the tTA transactivator (tetracycline repressor fused to the transcativation domain of protein VP16 from herpex simplex virus under the control of the human cytomegalovirus promotor) was introduced to SH-EP cells together with the pSV2neo plasmid conferring neomycin resistance. Clones selected in G418 were transiently transfected with plasmid pUHC13-3 (tetP-driven luciferase reporter construct) and analyzed for tetracycline-controlled expression of the luciferase gene. TetP is a synthetic promotor that consists of the CMV minimal promotor and tandem repeats of the tetracycline operator sequence (tetO). One of the clones, Tet-21, was co-transfected with the pHMR272 (hygromycin resistance) and the pUHD10-3/ N (tetP, MYCN cDNA and simian virus 40 polyadenylation signal) plasmids. Clones resistant to both neomycin and hygromycin were analyzed by immunoblotting for expression of MYCN in the presence (1 |g/ ml) and absence of tetracycline. This resulted to the generation of clone Tet-21/ N with high levels of MYCN induction in the absence of tetracycline.

### Example 9 Cation exchange chromatography of the SH-EP 007 conditioned medium

The concentrated and acidified medium was applied onto an S-Sepharose High Performance (SSHP) (20 ml bed volume) cation exchanger (Pharmacia) equilibrated in 50 mM acetate buffer, pH 4.8 + 150 mM NaCI + 0.1% CHAPS. Following application the column was eluted with (i) 3 bed volumes of the equilibration buffer, (ii) 5 bed volumes of 50 mM MES buffer, pH 6.0 + 150 mM NaCI + 0.1% CHAPS, (iii) 5 bed volumes of 50 mM Tris-HCI buffer, pH 7.2 + 150 mM NaCI + 0.1% CHAPS, and finally (iv) by 15 bed volumes of a linear gradient of buffer iii up to a salt concentration of 1 M NaCI. A flow rate of 60 cm/h was applied, and fractions of 4 ml were collected. Aliquots of the fractions were tested for protein content and activity on endothelial cell proliferation. The stimulatory activity was tested by adding 10-µl aliquots of the fractions on low density cultures of endothelial cells. A further 10-µl aliquot was added after two days and the cells were counted after 6 days of culture. The control wells received only buffer. The inhibitory activity was tested in the same manner except that all the wells received 2.5 ng/ml bFGF to stimulate the endothelial cells to a maximum. An aliquot of the flow though was also tested for activity in the same manner.

### Example 10 Cation exchange chromatography of the WAC 2 conditioned medium

The concentrated and acidified medium was chromatographed using an S-Sepharose High Performance (SSHP) (20 ml bed volume) cation exchanger (Pharmacia) as described in Example 9. Aliquots of the fractions were tested for protein content, stimulatory and inhibitory activity on endothelial (BBCE) cell proliferation as described in Example 9. An aliquot of the flow through was also tested for activity as described above.

### Example 11 Heparin - Sepharose chromatography of SS.1 and WI.2

The chromatography was carried out in 1 ml columns (0.8 cm diameter) equilibrated in 50 mM Tris-HCI, pH 7.2 + 150 mM NaCI + 0.1% CHAPS. The active fractions of Example 9 were concentrated and diafiltrated to the equilibration buffer using ultrafiltation. Following application of the sample, the column was eluted with (i) 5 bed volumes of equilibration buffer, (ii) a linear gradient consisting of 10 bed volumes of equilibation buffer and 10 bed volumes of 50 mM Tris-HCI, pH 7.2 + 2 M NaCI + 0.1% CHAPS, and (iii) 10 bed volumes of 50 mM Tris-HCI, pH 7.2 + 3 M NaCI + 0.1% CHAPS. A flow rate of 12 cm/h was used, and fractions of 1 ml volume were collected. Aliquots of the fractions were tested for protein content and stimulatory activity on endothelial (BBCE) cell proliferation.

### Example 12 Identification of TGFβ1

A) Aliquots of all partially purified inhibitors from SH-EP 007 and WAC 2 conditioned media were incubated with either chicken immunoglobulin or with chicken anti-TGFβ1 antibody (2.7 µg/ml). TGFβ1 (1.25 ng) was used as control to test the neutralization capacity of the antibody. Following incubation at room temperature for 30 min the samples and controls were tested for inhibtory activity on endothelial cell proliferation as described above. The activity is expressed as % control, that is the endothelial cell number after addition of bFGF and buffer only.
B) Immunodetection of partially purified fractions of SI.4 was achieved as follows. Aliquot of active fractions of SI.4 were electrophoresed on 13.5 % polyacrylamide gel without (lane 2) and with (lane 4) β-mercaptoethanol. In the same gel, TGFβ1 (50 ng) was electrophoresed also without (lane 1) and with (lane 3) β-mercaptoethanol. Following tranfer to nitrocellulose, immunodetection was carried out using rabbit anti-TGFβ1 antibody (1:1000) as the primary antibody followed by incubation with an anti-rabbit IgG labelled with peroxidase. Detection was carried out using an enhanced chemiluminescence system according to the instructions of the manufacturer *(ECL kit, Amersham, UK).* The sizes (kD) of the different bands of the marker are indicated with arrows on the left side.

## Claims

1. An isolated protein characterized in that its expression is downregulated by the expression of the oncogene MYCN and is not activin A.

2. An isolated protein according to claim 1 characterized in that it is capable of inhibiting endothelial cell growth.

3. An isolated protein according to any one of claims 1 and 2 characterized in that the protein has a molecular weight of about 47 kD when determined by gel filtration.

4. An isolated protein according to any one of claims 1 to 3 characterized in that it presents two peaks with isoelectric points of about 8.0 and 6.2 in chromatofocussing.

5. An isolated protein according to any one of claims 1 to 4 characterized in that it has no affinity to copper or heparin.

6. An isolated protein according to any one of claims 1 to 5 characterized in that it is glycosylated.

7. An isolated protein according to any one of claims 1 to 6 characterized in that it is heat stable and does not lose its inhibitory activity according to claim 2 after treatment at 90°C for 10 minutes.

8. An isolated protein according to any one of claims 1 to 7 characterized in that it is acid stable and does not lose its inhibitory activity according to claim 2 after treatment with 0.1 M HCl.

9. An isolated protein according to any one of claims 1 and 2 characterized in that:
- it has a molecular weight of about 47 kD when determined by gel filtration,
- it presents two peaks with isoelectric points of about 8.0 and 6.2 in chromatofocussing,
- it has no affinity to copper or heparin,
- it is glycosylated,
- it is heat stable and does not lose its inhibitory activity according to claim 2 after treatment at 90°C for 10 minutes,
- it is acid stable and does not lose its inhibitory activity according to claim 2 after treatment with 0.1 M HCI.

10. An isolated protein according to any one of claims 1 and 2 characterized in that the protein displays two molecular weights of about 47 and 29 kD when determined by gel filtration.

11. An isolated protein according to any one of claims 1, 2 and 10 characterized in that it presents two minor peaks and a main peak with an isoelectric point of about 5.0 in chromatofocussing.

12. An isolated protein according to any one of claims 1, 2 and 10 to 11 characterized in that it has no affinity to copper or heparin.

13. An isolated protein according to any one of claims 1, 2 and 10 to 12 characterized in that it is not glycosylated.

14. An isolated protein according to any one of claims 1, 2 and 10 to 13 characterized in that it is heat stable and does not lose its inhibitory activity according to claim 2 after treatment at 60°C for 60 minutes.

15. An isolated protein according to any one of claims 1, 2 and 10 to 14 characterized in that it is acid stable and does not lose its inhibitory activity according to claim 2 after treatment with 0.1 M HCI.

16. An isolated protein according to any one of claims 1 and 2 characterized in that:
- the polypeptide displays two molecular weights of about 47 and 29 kD when determined by gel filtration,
- it presents two minor and a main peak with an isoelectric point of about 5.0 in chromatofocussing,
- it has no affinity to copper or heparin,
- it is not glycosylated,
- it is heat stable and does not lose its inhibitory activity according to claim 2 after treatment at 60°C for 30 minutes,
- it is acid stable and does not lose its inhibitory activity according to claim 2 after treatment with 0.1 M HCl.

17. The functional derivative of any one of claims 1 to 16 wherein said functional derivative is a variant, a fragment, a chemical derivative, or a fusion protein of said protein.

18. A pharmaceutical composition comprising one or more proteins according to any one of claims 1 to 17 or pharmaceutically acceptable salts thereof.

19. The pharmaceutical composition of claim 18 for the treatment of pathological cellular growth, more particular pathological endothelial growth.

20. The pharmaceutical composition of claim 18 for the treatment of pathological angiogenic growth.

21. An antibody molecule specific for a protein according to any one of claims 1 to 17.

22. The antibody molecule of claim 21 characterized in that said antibody neutralises the biological activity of said polypeptide.

23. The antibody molecule according to any one of claims 21 or 22 which is a polyclonal antibody, a monoclonal antibody, a complete immunglobuline or a fragment thereof, the Fab' or F(ab)₂ fragment of an immunglobuline, a recombinant antibody or a recombinant antibody fragment, a recombinant single-chain antibody (scFv), a chimeric, bispecific or humanised antibody.

24. A process for preparing polyclonal antibodies according to any one of claims 21 to 23, characterized in that suitable host animals are immunized with a protein according to any one of claims 1 to 17 and polyclonal antibodies are isolated from said host animal.

25. A process for preparing monoclonal antibodies according to any one of claims 21 to 23, characterized in that suitable host animals are immunized with a protein according to any one of claims 1 to 17, B-lymphocytes of these host cells are fused with myeloma cells, and a hybrid cell line secreting the monoclonal antibody is subcloned and cultivated.

26. A process for preparing a hybrid cell line characterized in that it secretes monoclonal antibodies according to any one of claims 21 to 23.

27. A pharmaceutical composition comprising an antibody molecule according to any one of claims 21 to 23 or pharmaceutically acceptable salts thereof.

28. The pharmaceutical composition of claim 27 for the treatment of pathological cellular growth conditions, characterized in that normal endothelial cell growth is absent or pathologically reduced.

29. The pharmaceutical composition of claim 27 for the treatment of pathological conditions characterized in that normal angiogenic growth is absent or pathologically reduced.

30. The pharmaceutical composition of claim 27 characterized in that the pathological condition resulted from a wound.

31. A kit of parts containing in close confinement one or more carrier means, characterized in that one carrier means contains a monoclonal antibody according to any one of claims 25 to 27 for determining a polypeptide according to any one of claims 1 to 16.

32. The use of antibodies according to any one of claims 21 to 23 for purifying the polypeptides according to any one of claims 1 to 17.

33. The use of antibodies according to any one of claims 21 to 23 for qualitatively and/or quantitatively determining the polypeptides according to any one of claims 1 to 17 *in vitro* and *in vivo.*

34. The use of antibodies according to any one of claims 21 to 23 for neutralizing the activity of polypeptides according to any one of claims 1 to 16 *in vitro* and *in vivo.*

35. The use of antibodies according to any one of claims 21 to 23 for activating and/or enhancing cell growth, more particular endothelial cell growth *in vitro* and *in vivo.*

36. The use of antibodies according to any one of claims 21 to 23 for activating and/or enhancing angiogensis *in vitro* and *in vivo.*

37. The use of the proteins according to any one of claims 1 to 17 for inhibiting cell growth, more particular endothelial cell growth *in vitro* and *in vivo.*

38. The use of the proteins according to any one of claims 1 to 17 for inhibiting angiogenesis *in vitro* and *in vivo.*

39. The use of the proteins according to any one of claims 1 to 17 for inhibiting pathological cell growth, more particular pathological endothelial cell growth *in vitro* and *in vivo.*

40. The use of proteins according to any one of claims 1 to 17 for the preparation of a pharmaceutical composition.

41. The use of antibodies according to any one of claims 24 to 23 for the preparation of a pharmaceutical composition.

42. The use of proteins according to any one of claims 1 to 17 for the preparation of antibodies according to any one of claims 21 to 23.
